# EUROPÄISCHE PATENTSCHRIFT

(11) **EP 0 870 542 B1**
(45) Veröffentlichungstag und Bekanntmachung des Hinweises auf die Patenterteilung: **06.11.2002**
(21) Anmeldenummer: 98106482.7
(22) Anmeldetag: 08.04.1998
(51) Int. Cl.: B01J 23/00, B01J 23/04, C07C 2/72, C07C 2/54, C07C 5/25, C07C 2/24

(54) **Katalysator und Verfahren zur Seitenkettenalkylierung**
Side-chain alkylation process and catalyst
Catalyseur et procédé d'alkylation d'une chaîne latérale

(30) Priorität: 11.04.1997 DE 19715203
(43) Veröffentlichungstag der Anmeldung: 14.10.1998
(73) Patentinhaber: BASF AKTIENGESELLSCHAFT, 67056 Ludwigshafen (DE)
(72) Erfinder: Narbeshuber, Thomas Franz, Dr., 67069 Ludwigshafen (DE); Grosch, Georg Heinrich, Dr., 67098 Bad Dürkheim (DE); Trefzer, Michael, 67166 Otterstadt (DE); Gehrer, Eugen, Dr., 67069 Ludwigshafen (DE); Baier, Michael, Dr., 68161 Mannheim (DE)
(74) Vertreter: Isenbruck, Günter, Dr.

(56) Entgegenhaltungen:
- EP-A- 0 439 679
- WO-A-88/04956
- GB-A- 1 117 595
- GB-A- 1 550 873

## Beschreibung

Die Erfindung betrifft einen Katalysator, dessen Verwendung in stark basisch katalysierten Reaktionen und ein Verfahren zur Seitenkettenalkylierung oder Seitenkettenalkenylierung von alkylaromatischen oder alkylalicyclischen Verbindungen mit Olefinen oder Diolefinen.

Die Seitenkettenalkylierung, insbesondere von aromatischen Verbindungen, die ein acides Proton in der α-Position der Seitenkette tragen, in Gegenwart von Katalysatoren ist bekannt.

In der EP-B-0 439 679 ist ein Verfahren zur Alkylierung von alkylaromatischen Kohlenwasserstoffen beschrieben. Die Umsetzung erfolgt in Gegenwart eines Katalysators aus aktiviertem Aluminiumoxid, das mit Magnesiumhydroxid und Kaliummetall dotiert ist. Anstelle von Magnesiumhydroxid kamen auch Calciumhydroxid, Bariumhydroxid oder Magnesiumoxid zum Einsatz. Auch eine Imprägnierung mit Kaliumhydrid ist beschrieben.

US 4,914,250 betrifft ein Verfahren zur Seitenkettenalkylierung von Aromaten. Als Katalysator wurde dabei Diatomeenerde eingesetzt, die im Reaktionsgemisch neben Kalium oder NaK und Spuren von Wasser vorlag.

US 4,922,054 betrifft ebenfalls ein Verfahren zur Seitenkettenalkylierung von Aromaten, bei dem ebenfalls als Katalysator Diatomeenerde eingesetzt wurde, die im Reaktionsgemisch neben NaK und Kaliumoxid vorlag. Anstelle von Kaliumoxid wurde auch Rubidiumoxid verwendet. Anstelle von NaK wurde auch Kaliummetall eingesetzt.

JP-A2-05163171 betrifft die Herstellung von Alkenylbenzol und seinen Derivaten. Der verwendete Katalysator umfaßt ein Alkalimetall und ein Kaliumcarbonatsalz und/oder KOH, die in Gegenwart eines Olefins und/oder Diolefins dispergiert werden. Vorzugsweise wird als Alkalimetall Natriummetall und als Kaliumcarbonatsalz K₂CO₃, KHCO₃ oder KNaCO₃ eingesetzt.

GB-A-1 550 873 betrifft ein nicht oxidatives Dehydrierungsverfahren. Im Verfahren wird ein Spinellkatalysator eingesetzt, der durch ein oder mehrere Alkalimetalle aktiviert sein kann. Der im Beispiel hergestellte Katalysator wird mit Kaliumcarbonat aktiviert.

Diese bisher bekannten Katalysatoren sind in ihrer Leistung für viele Anwendungen unzureichend. Zum einen sind die bislang bekannten Katalysatoren wenig aktiv, das heißt die Raum-Zeit-Ausbeute ist sehr klein. Zudem bilden sie bei hohen Umsätzen und längeren Standzeiten unerwünschte Folgeprodukte der zunächst erhaltenen Primärprodukte. Beispielsweise kommt es bei der Seitenkettenalkylierung von Toluol mit Propen nach der Bildung von Isobutylbenzol zu Cyclisierungen zu Methylindan, wie auch zur Dimerisierung des Olefins, beispielsweise zur Bildung von Methylpenten aus Propen. Weiterhin ist die Standzeit der beschriebenen Katalysatoren begrenzt. Die Katalysatoren verlieren mit zunehmender Reaktionszeit ihre Aktivität und ändern teilweise ihr Nebenproduktespektrum.

Aufgabe der vorliegenden Erfindung ist die Bereitstellung eines Katalysators für die Seitenkettenalkylierung, der die Nachteile der bekannten Katalysatoren vermeidet und eine hohe Aktivität, Selektivität und Standzeit aufweist.

Die Aufgabe wird erfindungsgemäß gelöst durch Bereitstellung eines Katalysators, enthaltend mindestens ein metallisches Alkalimetall auf einem Spinell der allgemeinen Formel (I) oder Inversspinell der allgemeinen Formel (II) als Träger, der mit mindestens einer Verbindung eines Alkalimetalls und/oder Erdkalimetalls dotiert ist, wobei das Gewichtsverhältnis Alkalimetall/Träger 0,01 bis 5 und das Gewichtsverhältnis Dotierung/Träger 0,01 bis 5 beträgt.

Ferner wird die Aufgabe gelöst durch Verwendung dieses Katalysators in stark basisch katalysierten Reaktionen, vorzugsweise zur Seitenkettenalkylierung oder Seitenkettenalkenylierung von alkylaromatischen oder alkylalicyclischen Verbindungen mit Olefinen oder Diolefinen, zur Doppelbindungsisomerisierung von Olefinen oder zur Dimerisierung von Olefinen.

Die Aufgabe wird ferner beispielhaft gelöst durch Bereitstellung eines Verfahrens zur Seitenkettenalkylierung oder Seitenkettenalkenylierung von alkylaromatischen oder alkylalicyclischen Verbindungen durch Umsetzung mit Olefinen oder Diolefinen, wobei die Umsetzung in Gegenwart eines vorstehend definierten Katalysators durchgeführt wird.

Erfindungsgemäß wurde gefunden, daß auf den nachstehenden Spinellen oder Inversspinellen basierende Katalysatoren hervorragende Eigenschaften bei der Seitenkettenalkylierung zeigen.

Das Verhältnis Alkalimetall/Träger beträgt dabei vorzugsweise 0,01 bis 2, besonders bevorzugt 0,01 bis 1. Das Alkalimetall ist dabei vorzugsweise Natrium oder Kalium, insbesondere Natrium. Auch Gemische mehrerer Alkalimetalle können eingesetzt werden.

Das Alkalimetall liegt auf einem Spinell oder Inversspinell der allgemeinen Formeln (I) oder (II)

AB₂X₄ (I)

B(AB)X₄ (II)

mit der Bedeutung von
- A: zweiwertiges Metall,
- B: drei- oder vierwertiges Metall,
- X: O, S, Se, Halogen, Pseudohalogen
als Träger vor. Dabei sind die Ladungen ausgeglichen. Die als Träger eingesetzten Spinelle oder Inversspinelle sind an sich bekannt. Sie sind beispielsweise beschrieben in Holleman, Wiberg, Lehrbuch der anorganischen Chemie, Walter de Gruyter-Verlag, 101. Auflage, 1995, Seite 1061.

Vorzugsweise ist A ausgewählt aus Mg, Cr, Fe, Zn, Mn, Co, Ni, Cu, Cd, Sn und Gemischen davon, und B ist ausgewählt aus Al, Ga, In, Fe, V, Cr, Ti, Pb, Mn, Co, Rh, Ni, Si und Gemischen davon. X ist vorzugsweise O.

Der Träger ist dabei vorzugsweise ausgewählt aus Mg/Al-, Co/Al/Pb/Zn-, Co/Ni/Fe/Cr-, Co/Zn-, Cr/Zn/Co/Si-, Cu/Zn/Al- und Cu/Al-Spinellen.

Besonders bevorzugt sind Mg/Al- und Co/Al/Pb/Zn-Spinelle. In den Mg/Al-Spinellen beträgt vorzugsweise der Anteil an Al 20 bis 50, insbesondere 26 bis 45 und der Anteil an Mg 10 bis 20, insbesondere 14 bis 15 Gew.-%, der Anteil an Alkalimetall 3 bis 8, insbesondere 4,5 bis 6 Gew.-%, bezogen auf das Gesamtgewicht des Spinells. Im Co/Al/Pb/Zn-Spinell kommen vorzugsweise 15 bis 25 Gew.-% Co und jeweils 3 bis 6 Gew.-% Pb und Zn dazu. Der Träger ist ferner mit mindestens einer Verbindung eines Alkalimetalls und/oder Erdalkalimetalls im Gewichtsverhältnis Dotierung/Träger von 0,01 bis 5, vorzugsweise 0,01 bis 2, insbesondere 0,01 bis 1 dotiert. Die Dotierung der Träger erfolgt dabei vorzugsweise mit löslichen Verbindungen der Alkalimetalle und/oder Erdalkalimetalle, wie den Oxiden, Hydroxiden, Carbonaten, Formiaten, Acetaten, Oxalaten und/oder Hydriden. Bevorzugt werden die Hydroxide oder Carbonate, besonders bevorzugt K₂CO₃ und/oder KOH eingesetzt.

### Herstellung der Katalysatoren

Die Katalysatoren werden hergestellt durch
- Aufbringen mindestens eines Alkalimetalls auf den Träger der allgemeinen Formeln (I) oder (II) durch
   Trägerung des schmelzflüssigen Alkalimetalls, oder
   Imprägnierung oder Tränkung des Trägers mit Lösungen eines Alkalimetallazids, Trocknen des Trägers und Zersetzung des Alkalimetallazids, oder
   Aufdampfen des Alkalimetalls auf den Träger, oder
   Imprägnierung oder Tränkung des Trägers mit ammoniakalischen Lösungen des Alkalimetalls und Entfernen des Ammoniaks,
- wobei der Träger der allgemeinen Formeln (I) oder (II) vorher dotiert wurde durch Imprägnieren oder Tränken mit einer Lösung mindestens einer Verbindung eines Alkalimetalls und/oder Erdalkalimetalls, Trocknen und Calcinieren des dotierten Trägers.

Die Dotierung erfolgt dabei in an sich bekannter Weise durch Imprägnierung oder Tränkung und anschließende Calcinierung bei Temperaturen im Bereich von 200 bis 1500°C, vorzugsweise 250 bis 1000°C, besonders bevorzugt 250 bis 900°C. Dabei kann die Imprägnierung oder Tränkung mit einer Lösung der Verbindung des Alkalimetalls und/oder Erdalkalimetalls in einem beliebigen geeigneten Lösungsmittel erfolgen. Vorzugsweise werden wäßrige Lösungen eingesetzt, wobei nach dem Imprägnieren oder Tränken das Wasser durch Trocknen des imprägnierten Trägers entfernt wird. Es kann auch ohne vorherige Trocknung calciniert werden, wobei zu Beginn der Calcinierung das Lösungsmittel entweicht. Die Calcinierung des dotierten Trägers kann unter vermindertem Druck, unter Normaldruck oder unter erhöhtem Druck durchgeführt werden. Sie kann dabei sowohl in sauerstoffhaltiger Atmosphäre, als auch in Inertgasatmosphäre, wie unter Helium, Stickstoff oder Argon, oder unter Reaktivgasatmosphäre, wie unter Wasserstoff, Ammoniak, Kohlendioxid oder Kohlenmonoxid stattfinden.

Die Alkalimetalle werden in an sich bekannter Weise auf die, vorzugsweise dotierten, Träger aufgebracht. Hierzu gehört die Trägerung im schmelzflüssigen Zustand bei einer Temperatur im Bereich von 100 bis 300°C, wie sie beispielsweise in GB-A-1 143 993 beschrieben ist. Dazu wird die entsprechende Menge des Alkalimetalls als Strang oder Block zum Träger gegeben und unter Erwärmen mit ihm vermischt. Dabei erfolgt die feine Verteilung des Alkalimetalls auf dem Träger. Ferner können die Alkalimetalle durch Imprägnierung mit Lösungen der Alkalimetallazide und anschließende thermische Zersetzung der Azide hergestellt werden. Ein entsprechendes Verfahren ist beispielsweise in FR-A-2 609 024 beschrieben. Weiterhin können die Alkalimetalle durch Aufdampfen auf den Träger aufgebracht werden. Dies geschieht in der Regel unter vermindertem Druck.

Weiterhin können die Träger mit ammoniakalischen Lösungen der Alkalimetalle imprägniert werden, und der Ammoniak kann anschließend verflüchtigt werden. Die Trägerung der Alkalimetalle findet dabei vorzugsweise unter vermindertem Druck oder unter Inertgasatmosphäre, wie unter Helium, Stickstoff, Wasserstoff oder Argon statt.

Die Katalysatoren werden in stark basisch katalysierten Reaktionen, vorzugsweise zur Seitenkettenalkylierung oder Seitenkettenalkenylierung von alkylaromatischen oder alkylalicyclischen Verbindungen mit Olefinen oder Diolefinen, zur Doppelbindungsisomerisierung von Olefinen oder zur Dimersisierung von Olefinen eingesetzt.

Dabei wird die Umsetzung im allgemeinen bei einer Temperatur von -50 bis 400°C, vorzugsweise bei einer Temperatur von -20 bis 300°C, besonders bevorzugt 80 bis 250°C, insbesondere 100 bis 220°C und einem Druck von vorzugsweise 0,1 bis 200, besonders bevorzugt 1 bis 150, insbesondere 1 bis 100 bar durchgeführt.

Als alkylaromatische Verbindungen können dabei alle geeigneten Alkylaromaten eingesetzt werden. Sie können als aromatischen Kern beispielsweise einen Benzol- oder Naphthalinkern aufweisen. Weiterhin sind alkylalicyclische Verbindungen geeignet, in denen der cyclische Kern ein cyclischer Alkyl-, Alkenyl- oder Alkinylrest sein kann. Auch Reste, in denen mehrere der Ringstrukturen miteinander verknüpft sind, können eingesetzt werden. Die Ringstrukturen weisen in α-Position der Seitenkette ein azides Wasserstoffatom auf. Vorzugsweise weisen sie mindestens einen Alkylrest auf, der an die cyclische Struktur gebunden ist. Die Alkylreste können dabei eine beliebige Länge aufweisen und durch weitere Substituenten substituiert sein. Vorzugsweise werden als alkylaromatische Verbindungen Benzole, die durch 1 bis 6, vorzugsweise 1 bis 3, insbesondere 1 bis 2 C₁₋₂₀-, vorzugsweise C₁₋₃-Alkylreste substituiert sind, Naphthaline, die durch 1 bis 10, vorzugsweise 1 bis 5, besonders bevorzugt 1 bis 2 C₁₋₂₀-, vorzugsweise C₁₋₃-Alkylreste substituiert sind, und als alkylalicyclische Verbindungen Cyclopentene oder Cyclohexene eingesetzt, die durch 1 bis 5, vorzugweise 1 oder 2 beziehungsweise 1 bis 6, vorzugsweise 1 bis 3, insbesondere 1 oder 2 C₁₋₂₀-, vorzugsweise C₁₋₃-Alkylreste substituiert sind.

Die Olefine weisen vorzugsweise 2 bis 20, besonders bevorzugt 2 bis 10, insbesondere 2 bis 5 C-Atome auf. Vorzugsweise werden Ethen, Propen, 1-Buten, 2-Buten, Isobuten, 1-Penten, 2-Penten, 2-Methyl-1-buten, 2-Methyl-2-buten und/oder 3-Methyl-1-buten eingesetzt. Besonders bevorzugt sind Ethen und Propen. Die Diolefine weisen vorzugsweise 4 bis 20, besonders bevorzugt 4 bis 10, insbesondere 4 bis 6 C-Atome auf. Besonders bevorzugt werden Butadien und/oder Isopren eingesetzt.

Besonders bevorzugt sind die Umsetzung von Toluol mit Ethen oder Propen zu Propylbenzol oder Isobutylbenzol, die Umsetzung von Cumol mit Ethen zu tert.-Amylbenzol und die Umsetzung von Xylolen mit Butadien zu 5-Tolylpentenen.

Die Umsetzung kann diskontinuierlich oder vorzugsweise kontinuierlich in der Flüssig- oder Gasphase, bevorzugt in der Flüssigphase durchgeführt werden. Dabei können die bekannten Vorrichtungen zur Durchführung des Verfahrens eingesetzt werden.

Nachstehend wird die Erfindung anhand von Beispielen weiter erläutert.

### Beispiele

### Herstellungsbeispiele

Am Ende jedes Beispiels sind die Elementaranalysen pro 100 g des dotierten Trägers vor der Aufbringung des Alkalimetalls in Klammern angegeben.

### Katalysator A (Vergleich)

γ-Al₂O₃ wurde mit 10 Gew.-% K₂CO₃ (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 44,5 g; K 5,9 g).

### Katalysator B

Mg/Al-Spinell von Baikowski, Nancy, Frankreich wurde mit 10 Gew.-% K₂CO₃ (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 33 g; Mg 14,6 g; K 5g).

### Katalysator C

Mg/Al Spinell (Baikowski) wurde mit 10 Gew.-% NaOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Kalium zugefügt und bei 300°C dispergiert (Al 34 g; Mg 14,6 g; Na 5,7 g).

### Katalysator D

Mg/Al Spinell (Baikowski) wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Kalium zugefügt und bei 300°C dispergiert (Al 35 g; Mg 14,7 g; K 4,5 g).

### Katalysator E

Mg/Al Spinell (Baikowski) wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 35 g; Mg 14,7 g; K 4,5 g).

### Katalysator F

Mg/Al Spinell (Baikowski) wurde mit 10 Gew.-% CsNO₃ (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 36,5 g; Mg 14,8 g; Cs 5 g).

### Katalysator G

Co/Al/Pb/Zn-Spinell (Hersteller BASF AG, Ludwigshafen) wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 26,5 g; Co 20,3 g; K 5,5 g; Pb 4,6 g: Zn 4,6 g).

### Katalysator H

Co/Ni/Fe/Cr Spinell (BASF) wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Fe 24 g; Cr 23,6 g; Co 6,8 g; Ni 9,4 g; K 5,7 g).

### Katalysator J

Co/Zn Spinell (BASF) wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Zn 59 g; Co 10 g; K 5,4 g).

### Katalysator K

Co/Zn/Cr/Si-Spinell (BASF) wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Cr 37 g; Zn 10,5 g; Co 6,9 g; Si 6,2 g; K 5,6 g).

### Katalysator L

Cu/Zn/Al Spinell wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 29,4 g; Cu 10,6 g; Zn 13,8 g; K 5,4 g).

### Katalysator M

Cu/Al Spinell wurde mit 10 Gew.-% KOH (gelöst in H₂O) imprägniert. Die Suspension wurde zur Trockene eingedampft und das so erhaltene Pulver bei 500°C im Luftstrom kalziniert. Das Material wurde dann im Vakuum bei 300°C 3 h lang trocken gerührt. Zu diesem Pulver wurden 10 Gew.-% metallisches Natrium zugefügt und bei 300°C dispergiert (Al 36 g; Cu 13,4 g; K 4,6 g).

### Katalysator N

Es wurde der gleiche Spinell wie für Katalysator D eingesetzt. Die Kalzinierung fand jedoch bei 250°C statt.

### Katalysator O

Es wurde der gleiche Spinell wie für Katalysator E eingesetzt. Die Kalzinierung fand jedoch bei 250°C statt.

### Katalysator P

Es wurde der gleiche Spinell wie für Katalysator E eingesetzt. Die Kalzinierung fand jedoch bei 600°C statt.

### Katalysator Q

Es wurde der gleiche Spinell wie für Katalysator E eingesetzt. Die Kalzinierung fand jedoch bei 700°C statt.

### Katalysator R

Es wurde der gleiche Spinell wie für Katalysator E eingesetzt. Die Kalzinierung fand jedoch bei 800°C statt.

### Verfahrensbeispiele

### Vergleichsbeispiel V1

10 g Katalysator A wurden mit 85 g Toluol in einem druckfesten Reaktionsgefäß vorgelegt. Nach Zugabe von 20 g Propen wurde das Reaktionsgefäß auf 160°C aufgeheizt und die Reaktionssuspension anschließend 12 h lang gerührt. Die Ergebnisse sind in Tabelle 1 aufgeführt.

### Beispiele 1 - 16

10 g der Katalysatoren B bis R wurden jeweils mit 85 g Toluol in einem druckfesten Reaktionsgefäß vorgelegt. Nach Zugabe von 20 g Propen wurde das Reaktionsgefäß auf 160°C aufgeheizt und die Reaktionssuspension anschließend 12 h lang gerührt. Die Ergebnisse sind in Tabelle 1 aufgeführt.

**Tabelle 1**

| | Katalysator | U_{Propen} | S_{iBB} | S_{BB} | MP | MI |
|---|---|---|---|---|---|---|
| Vergleichsbeispiel V1 | A | 34% | 66% | 70% | 5,7% | 0,2% |
| Beispiel 1 | B | 42% | 77% | 86% | 2,6% | 1,5% |
| Beispiel 2 | C | 37% | 81% | 86% | 1,6% | 1,1% |
| Beispiel 3 | D | 50% | 80% | 89% | 2,6% | 0,1% |
| Beispiel 4 | E | 22% | 73% | 79% | 1,9% | 0,7% |
| Beispiel 5 | F | 16% | 78% | 88% | 1,0% | 0,2% |
| Beispiel 6 | G | 60% | 78% | 87% | 3,3% | 0,3% |
| Beispiel 7 | H | 30% | 80% | 89% | 1,5% | 0,1% |
| Beispiel 8 | J | 20% | 71% | 81% | 1,8% | 0,1% |
| Beispiel 9 | K | 13% | 72% | 79% | 1,4% | 0,4% |
| Beispiel 10 | L | 35% | 74% | 82% | 3,3% | 0,1% |
| Beispiel 11 | M | 22% | 68% | 75% | 2,4% | 0,1% |
| Beispiel 12 | N | 50% | 81% | 90% | 2,4% | 0,1% |
| Beispiel 13 | O | 28% | 77% | 82% | 1,6% | 1,2% |
| Beispiel 14 | P | 20% | 80% | 85% | 1,0% | 0,8% |
| Beispiel 15 | Q | 35% | 74% | 79% | 2,3% | 1,7% |
| Beispiel 16 | R | 34% | 74% | 79% | 2,6% | 1,4% |
| UPropen = Umsatz Propen [mol%] SiBB = Selektivität für Isobutylbenzol [mol%] S_{BB} = Selektivität für Iso- und n-Butylbenzol [mol%] MP = Methylpenten, Gew.-% im Austrag MI = Methylindan, Gew.-% im Austrag | | | | | | |

### Vergleichsbeispiel V2

20 g Katalysator wurden in einem druckfesten Reaktionsgefäß (Volumen 150 ml) mit 100 g Toluol vorgelegt und auf 160°C aufgeheizt. Danach wurde eine Mischung aus Toluol und Propen (Mol.-Verhältnis Toluol: Propen = 2:1) kontinuierlich (6 ml/h) in das Reaktionsgefäß dosiert. Der Druck wurde bei 50 bar gehalten. Tabelle 2 gibt die Ergebnisse wieder. Der Austrag an Methylpenten stieg kontinuierlich an und fand bis zum Abbruch der Reaktion kein Ende.

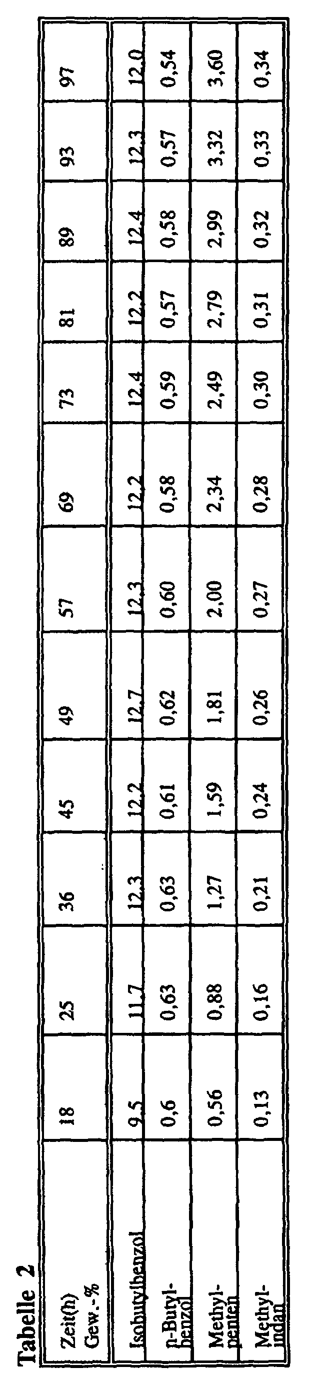

### Beispiel 12

20 g Katalysator B wurden in einem druckfesten Reaktionsgefäß (Volumen 150 ml) mit 100 g Toluol vorgelegt und auf 160°C aufgeheizt. Danach wurde eine Mischung aus Toluol und Propen (Mol.-Verhältnis Toluol: Propen = 2:1) kontinuierlich (6 ml/h) in das Reaktionsgefäß dosiert. Der Druck wurde bei 50 bar gehalten. Tabelle 3 gibt Ergebnisse wieder. Die Ausbeute an Isobutylbenzol war größer und die Menge der gebildeten Nebenprodukte bedeutend geringer als beim Vergleichsbeispiel V2.

**Tabelle 3**

| Zeit(h) Gew.-% | 49 | 54 | 62 | 70 | 78 | 86 | 98 | 106 | 126 | 130 | 138 | 148 | 153 | 161 |
|---|---|---|---|---|---|---|---|---|---|---|---|---|---|---|
| Isobutylbenzol | 10,1 | 13,0 | 14,8 | 15,9 | 17,4 | 18,6 | 19,4 | 21,8 | 23,2 | 26,9 | 26,8 | 25,70 | 26,00 | 25,40 |
| n-Butylbenzol | 0,74 | 0,90 | 0,98 | 1,02 | 1,07 | 1,11 | 1,12 | 1,25 | 1,30 | 1,48 | 1,46 | 1,40 | 1,42 | 1,39 |
| Methylpenten | 0,49 | 0,54 | 0,74 | 0,85 | 0,98 | 1,18 | 1,40 | 1,42 | 1,75 | 2,24 | 2,14 | 2,21 | 2,32 | 2,37 |
| Methylindan | 0,6 | 0,9 | 1,0 | 1,1 | 1,3 | 1,4 | 1,5 | 1,8 | 1,8 | 2,1 | 2,1 | 2,0 | 2,0 | 2,0 |

## Patentansprüche

1. Katalysator, enthaltend mindestens ein metallisches Alkalimetall auf einem Spinell oder Inversspinell als Träger, der mit mindestens einer Verbindung eines Alkalimetalls und/oder Erdkalimetalls dotiert ist, wobei das Gewichtsverhältnis Alkalimetall/Träger 0,01 bis 5 und das Gewichtsverhältnis Dotierung/Träger 0,01 bis 5 beträgt und der Spinell die allgemeine Formel (I) und der Inversspinell die allgemeine Formel (II)
AB₂X₄ (I)
B(AB)X₄ (II)
mit der Bedeutung von
A zweiwertiges Metall,
B drei- oder vierwertiges Metall,
X O, S, Se, Halogen, Pseudohalogen
aufweist, wobei die Ladungen ausgeglichen sind, wobei vorzugsweise A ausgewählt ist aus Mg, Cr, Fe, Zn, Mn, Co, Ni, Cu, Cd, Sn und Gemischen davon, und B ausgewählt ist aus Al, Ga, In, Fe, V, Cr, Ti, Pb, Mn, Co, Rh, Ni, Si und Gemischen davon, und X O ist.

2. Katalysator nach Anspruch 1, **dadurch gekennzeichnet, daß** der Träger ausgewählt ist aus Mg/Al-, Co/Al/Pb/Zn-, Co/Ni/Fe/Cr-, Co/Zn-, Cr/Zn/Co/Si-, Cu/Zn/Al- und Cu/Al-Spinellen.

3. Verfahren zur Herstellung von Katalysatoren gemäß einem der Ansprüche 1 oder 2 durch
- Aufbringen mindestens eines Alkalimetalls auf den Träger der allgemeinen Formeln (I) oder (II) durch
Trägerung des schmelzflüssigen Alkalimetalls, oder
Imprägnierung oder Tränkung des Trägers mit Lösungen eines Alkalimetallazids, Trocknen des Trägers und Zersetzung des Alkalimetallazids, oder
Aufdampfen des Alkalimetalls auf den Träger, oder
Imprägnierung oder Tränkung des Trägers mit ammoniakalischen Lösungen des Alkalimetalls und Entfernen des Ammoniaks,
- wobei der Träger der allgemeinen Formeln (I) oder (II) vorher dotiert wurde durch Imprägnieren oder Tränken mit einer Lösung mindestens einer Verbindung eines Alkalimetalls und/oder Erdalkalimetalls, Trocknen und Calcinieren des dotierten Trägers.

4. Verwendung eines Katalysators nach einem der Ansprüche 1 oder 2 in stark basisch katalysierten Reaktionen, vorzugsweise zur Seitenkettenalkylierung oder Seitenkettenalkenylierung von alkylaromatischen oder alkylalicyclischen Verbindungen mit Olefinen oder Diolefinen, zur Doppelbindungsisomerisierung von Olefinen oder zur Dimerisierung von Olefinen.

5. Verfahren zur Seitenkettenalkylierung oder Seitenkettenalkenylierung von alkylaromatischen oder alkylalicyclischen Verbindungen durch Umsetzung mit Olefinen oder Diolefinen, **dadurch gekennzeichnet, daß** die Umsetzung in Gegenwart eines Katalysators durchgeführt wird, wie er in einem der Ansprüche 1 oder 2 definiert ist.

6. Verfahren nach Anspruch 5, **dadurch gekennzeichnet, daß** die Umsetzung bei einer Temperatur im Bereich von -50 bis 400°C und einem Druck im Bereich von 0,1 bis 200 bar durchgeführt wird.

7. Verfahren nach einem der Ansprüche 5 oder 6, **dadurch gekennzeichnet, daß** als alkylaromatische Verbindungen Benzole, die durch 1 bis 6 C₁₋₂₀-Alkylreste substituiert sind, Naphthaline, die durch 1 bis 10 C₁₋₂₀-Alkylreste substituiert sind, und als alkylalicyclische Verbindungen Cyclopentene oder Cyclohexene, die durch 1 bis 5 beziehungsweise 1 bis 6 C₁₋₂₀-Alkylreste substituiert sind, eingesetzt werden.

8. Verfahren nach einem der Ansprüche 5 bis 7, **dadurch gekennzeichnet, daß** als Olefine Ethen, Propen, 1-Buten, 2-Buten, Isobuten, 1-Penten, 2-Penten, 2-Methyl-1-buten, 2-Methyl-2-buten und/oder 3-Methyl-1-buten, und als Diolefine Butadien oder Isopren eingesetzt werden.

9. Verfahren nach einem der Ansprüche 5 bis 8, **dadurch gekennzeichnet, daß** es in der Flüssig- oder Gasphase kontinuierlich durchgeführt wird.

## Claims

1. A catalyst comprising at least one metallic alkali metal on a spinel or inverse spinel as carrier, which is doped with at least one compound of an alkali metal and/or alkaline earth metal, where the alkali metal/carrier ratio by weight is from 0.01 to 5 and the dopant/carrier ratio by weight is from 0.01 to 5 and the spinel has formula (I) and the inverse spinel has formula (II)
AB₂X₄ (I)
B(AB)X₄ (II)
where
A is divalent metal,
B is tri- or tetravalent metal,
X is O, S, Se, halogen, pseudohalogen,
the charges being balanced, and A preferably being selected from Mg, Cr, Fe, Zn, Mn, Co, Ni, Cu, Cd, Sn and mixtures thereof, and B being selected from Al, Ga, In, Fe, V, Cr, Ti, Pb, Mn, Co, Rh, Ni, Si and mixtures thereof, and X being O.

2. A catalyst as claimed in claim 1, wherein the carrier is selected from Mg/Al, Co/Al/Pb/Zn, Co/Ni/Fe/Cr, Co/Zn, Cr/Zn/Co/Si, Cu/Zn/Al and Cu/Al spinels.

3. A process for preparing a catalyst as claimed in either of claims 1 and 2 by
- applying at least one alkali metal to the carrier of the formulae (I) or (II) by
applying the molten alkali metal to the carrier or
impregnating the carrier with solutions of an alkali metal azide, drying the carrier and decomposing the alkali metal azide or
vapor-depositing the alkali metal on the carrier or
impregnating the carrier with ammoniacal solutions of the alkali metal and removing the ammonia,
- where the carrier of the formulae (I) or (II) has been previously doped by impregnating with a solution of at least one compound of an alkali metal and/or alkaline earth metal, drying and calcining the doped carrier.

4. The use of a catalyst as claimed in either of claims 1 and 2 in reactions catalyzed by strong bases, preferably for the side-chain alkylation or side-chain alkenylation of alkylaromatic or alkylalicyclic compounds with olefins or diolefins, for double-bond isomerization of olefins or for dimerization of olefins.

5. A process for the side-chain alkylation or side-chain alkenylation of alkylaromatic or alkylalicyclic compounds by reaction with olefins or diolefins, wherein the reaction is carried out in the presence of a catalyst as defined in either of claims 1 and 2.

6. A process as claimed in claim 5, wherein the reaction is carried out at from -50 to 400°C under a pressure of from 0.1 to 200 bar.

7. A process as claimed in either of claims 5 or 6, wherein the alkylaromatic compounds employed are benzenes substituted by 1 to 6 C₁₋₂₀ alkyl radicals or naphthalenes substituted by 1 to 10 C₁₋₂₀ alkyl radicals, and the alkylalicyclic compounds employed are cyclopentenes or cyclohexenes respectively substituted by 1 to 5 or 1 to 6 C₁₋₂₀ alkyl radicals.

8. A process as claimed in any of claims 5 to 7, wherein the olefins employed are ethene, propene, 1-butene, 2-butene, isobutene, 1-pentene, 2-pentene, 2-methyl-1-butene, 2-methyl-2-butene and/or 3-methyl-1-butene, and the diolefins employed are butadiene or isoprene.

9. A process as claimed in any of claims 5 to 8, which is carried out continuously in the liquid or gas phase.

## Revendications

1. Catalyseur contenant au moins un métal alcalin métallique déposé sur un spinelle ou sur un spinelle inverse à titre de support qui a été dopé avec au moins un composé d'un métal alcalin et/ou d'un métal alcalino-terreux, le rapport pondéral métal alcalin/support s'élevant de 0,01 à 5 et le rapport pondéral dopant/support s'élevant de 0,01 à 5, le spinelle répondant à la formule générale (I) et le spinelle inverse répondant à la formule générale (II)
AB₂X₄ (I)
B(AB)X₄ (II)
formules dans lesquelles
A représente un métal divalent,
B représente un métal trivalent ou tétravalent,
X représente un atome d'oxygène, un atome de soufre, un atome de sélénium, un atome d'halogène, un pseudohalogène,
dans lequel les charges ont été équilibrées, dans lequel de préférence A est choisi parmi le groupe constitué par Mg, Cr, Fe, Zn, Mn, Co, Ni, Cu, Cd, Sn et leurs mélanges, et B est choisi parmi le groupe constitué par Al, Ga, In, Fe, V, Cr, Ti, Pb, Mn, Co, Rh, Ni, Si et leurs mélanges, et X représente un atome d'oxygène.

2. Catalyseur selon la revendication 1, **caractérisé en ce que** le support est choisi parmi des spinelles de Mg/Al, Co/Al/Pb/Zn, Co/Ni/Fe/Cr, Co/Zn, Cr/Zn/Co/Si, Cu/Zn/Al et Cu/Al.

3. Procédé pour la préparation de catalyseurs selon l'une quelconque des revendications 1 ou 2, par
- application d'au moins un métal alcalin sur le support répondant à la formule générale (I) ou (II) par
déposition sur le support du métal alcalin en fusion, ou
imprégnation ou imbibition du support avec des solutions d'un azide de métal alcalin, séchage du support et décomposition de l'azide de métal alcalin, ou
vaporisation du métal alcalin sur le support, ou
imprégnation ou imbibition du support avec des solutions ammoniacales du métal alcalin et élimination de l'ammoniac,
- dans lequel le support répondant à la formule générale (I) ou (II) a été dopé au préalable par imprégnation ou par imbibition avec une solution d'au moins un composé d'un métal alcalin et/ou d'un métal alcalino-terreux, séchage et calcination du support dopé.

4. Utilisation d'un catalyseur selon l'une quelconque des revendications 1 ou 2, dans des réactions catalysées à l'aide d'un catalyseur fortement basique, de préférence pour l'alkylation des chaînes latérales ou pour l'alcénylation des chaînes latérales de composés alkylaromatiques ou alkylalicycliques avec des oléfines ou des dioléfines, pour l'isomérisation de liaisons doubles d'oléfines ou encore pour la dimérisation d'oléfines.

5. Procédé pour l'alkylation des chaînes latérales ou pour l'alcénylation des chaînes latérales de composés alkylaromatiques ou alkylalicycliques par mise en réaction avec des oléfines ou des dioléfines, **caractérisé en ce qu'**on effectue la mise en réaction en présence d'un catalyseur tel qu'il a été défini dans l'une quelconque des revendications 1 ou 2.

6. Procédé selon la revendication 5, **caractérisé en ce qu'**on effectue la mise en réaction à une température dans la plage de -50 à 400 °C et sous une pression dans la plage de 0,1 à 200 bar.

7. Procédé selon l'une quelconque des revendications 5 ou 6, **caractérisé en ce qu'**on met en oeuvre, à titre de composés alkylaromatiques, des benzènes qui sont substitués par 1 à 6 radicaux alkyle en C₁-C₂₀, des naphtalènes qui sont substitués par 1 à 10 radicaux alkyle en C₁-C₂₀, et à titre de composés alkylalicycliques, des cyclopentènes ou des cyclohexènes qui sont substitués par 1 à 5, respectivement 1 à 6 radicaux alkyle en C₁-C₂₀.

8. Procédé selon l'une quelconque des revendications 5 à 7, **caractérisé en ce qu'**on met en oeuvre à titre d'oléfines, l'éthène, le propène, le 1-butène, le 2-butène, l'isobutène, le 1-pentène, le 2-pentène, le 2-méthyl-1-butène, le 2-méthyl-2-butène et/ou le 3-méthyl-1-butène, et à titre de dioléfines, le butadiène ou l'isoprène.

9. Procédé selon l'une quelconque des revendications 5 à 8, **caractérisé en ce qu'**on le met en oeuvre en continu en phase liquide ou en phase gazeuse.
